# EUROPEAN PATENT APPLICATION

(11) **EP 4 542 565 A1**
(43) Date of publication of application: **23.04.2025**
(21) Application number: 24203924.6
(22) Date of filing: 01.10.2024
(51) Int. Cl.: G16H 30/20, G16H 30/40, G16H 40/63

(54) **SYSTEMS AND METHODS FOR DETERMINING A DATA QUALITY OF DIGITAL IMAGING AND COMMUNICATIONS IN MEDICINE (DICOM) DATA**

(30) Priority: 16.10.2023 US 202318487341
(71) Applicant: GE Precision Healthcare LLC, Waukesha, WI 53188 (US)
(72) Inventor: BEDEZ, Mathieu, 67000 Strasbourg (FR); CALDINI QUEIROS, Celine, 67000 Strasbourg (FR); GERNER, Philippe, 67000 Strasbourg (FR)
(74) Representative: Kilburn & Strode LLP

(57) **Abstract**

A system (100) and method (300) for determining a data quality of DICOM data (412, 416, 420) are provided. Information related to a data ingestion (402) of the DICOM data (412, 416, 420) from a radiology device (102) to a database (110) may be received (302). Respective data qualities of the DICOM data (412, 416, 420) at the set of data ingestion stages (404, 406, 408, 410) of the data ingestion (402) may be determined (304) using a set of data validation rules (414, 418, 422) corresponding to a set of data ingestion stages (404, 406, 408, 410) of the data ingestion (402) of the DICOM data (412, 416, 420) from the radiology device (102) to the database (110). A user interface (700, 800) that displays information related to the respective data qualities of the DICOM data (412, 416, 420) at the set of data ingestion stages (404, 406, 408, 410) of the data ingestion (402) may be generated (306).

## Description

### TECHNICAL FIELD

The present disclosure relates, generally, to systems and methods for determining a data quality of DICOM data. More specifically, the present disclosure is related to systems and methods for determining data quality of DICOM data at a set of data ingestion stages of a data ingestion of the DICOM data from a radiology device to a database.

### BACKGROUND

In modern healthcare systems, data is increasingly important. This may be device data, patient data, treatment data, imaging scan data, and other sources of healthcare related data. The quality and usefulness of the data is important for efficient, cost effective, and accurate healthcare decisions. Data quality may refer to a measure of a condition of data that identifies a fitness of the data for a particular use. For example, a data quality of data may identify an accuracy, a completeness, a comprehensiveness, a consistency, a timeliness, a uniqueness, a validity, etc., of the data. In a medical context, a system of a health care institution (e.g., a hospital, a clinic, a care facility, or the like) may perform data ingestion of DICOM data from radiology devices that generate the DICOM data to a database that stores the DICOM data. The data quality of the DICOM data may vary at different data ingestion stages of the data ingestion, and/or might be affected at various data ingestion stages of the data ingestion. For instance, the DICOM data might have a low data quality at a data generation stage based on an incorrect configuration of the radiology device that is configured to generate the DICOM data. As another example, the DICOM data might have a data quality that is impacted during a data extraction stage based on network connectivity issues of the healthcare institution. As another example, the DICOM data might have a data quality that is reduced during a data processing stage based on an incorrect configuration of a data ingestion application that processes the DICOM data. The healthcare institution might have a large number of radiology devices. Personnel of the hospital institution might attempt to assess the data quality of DICOM data of the radiology devices. However, this task might prove impossible, inefficient, error-prone, or the like.

### SUMMARY

This summary introduces concepts that are described in more detail in the detailed description. It should not be used to identify essential features of the claimed subject matter, nor to limit the scope of the claimed subject matter.

In an aspect, a method may include receiving information related to a data ingestion of Digital Imaging and Communications in Medicine (DICOM) data from a radiology device to a database; determining, using a set of data validation rules corresponding to a set of data ingestion stages of the data ingestion of the DICOM data from the radiology device to the database, respective data qualities of the DICOM data at the set of data ingestion stages of the data ingestion, wherein the data validation rules include one or more of a first data validation rule that is generic to a plurality of imaging modalities of radiology devices, a second data validation rule that is specific to an imaging modality of the radiology device, and a third data validation rule that is specific to a data ingestion application associated with the data ingestion; and generating a user interface that displays information related to the respective data qualities of the DICOM data at the set of data ingestion stages of the data ingestion based on determining the respective data qualities of the DICOM data at the set of data ingestion stages of the data ingestion.

In another aspect, a device may include a memory configured to store instructions; and one or more processors configured to execute the instructions to: receive information related to a data ingestion of Digital Imaging and Communications in Medicine (DICOM) data from a radiology device to a database; determine, using a set of data validation rules corresponding to a set of data ingestion stages of the data ingestion of the DICOM data from the radiology device to the database, respective data qualities of the DICOM data at the set of data ingestion stages of the data ingestion, wherein the data validation rules include a first data validation rule that is generic to a plurality of imaging modalities of radiology devices, a second data validation rule that is specific to an imaging modality of the radiology device, and a third data validation rule that is specific to a data ingestion application associated with the data ingestion; and generate a user interface that displays information related to the respective data qualities of the DICOM data at the set of data ingestion stages of the data ingestion based on determining the respective data qualities of the DICOM data at the set of data ingestion stages of the data ingestion.

In yet another aspect, a non-transitory computer-readable medium may store instructions that, when executed by one or more processors, cause the one or more processors to: receive information related to a data ingestion of Digital Imaging and Communications in Medicine (DICOM) data from a radiology device to a database; determine, using a set of data validation rules corresponding to a set of data ingestion stages of the data ingestion of the DICOM data from the radiology device to the database, respective data qualities of the DICOM data at the set of data ingestion stages of the data ingestion, wherein the data validation rules include a first data validation rule that is generic to a plurality of imaging modalities of radiology devices, a second data validation rule that is specific to an imaging modality of the radiology device, and a third data validation rule that is specific to a data ingestion application associated with the data ingestion; and generate a user interface that displays information related to the respective data qualities of the DICOM data at the set of data ingestion stages of the data ingestion based on determining the respective data qualities of the DICOM data at the set of data ingestion stages of the data ingestion.

Some embodiments of the present disclosure provide a technical improvement in the technical field of radiology and a technical improvement to devices associated with the acquisition, ingestion, and usage of DICOM data of radiology devices by determining data quality of DICOM data at a set of data ingestion stages of a data ingestion of the DICOM data.

### BRIEF DESCRIPTION OF DRAWINGS

FIG. 1 is a diagram of an example system for determining a data quality of DICOM data.
FIG. 2 is a diagram of example components of a device of FIG. 1.
FIG. 3 is a diagram of a flowchart of an example process for determining a data quality of DICOM data using a set of validation rules corresponding to a set of data ingestion stages of a data ingestion of the DICOM data from a radiology device to a database.
FIG. 4 is a diagram of an example process for determining a data quality of DICOM data using a set of validation rules corresponding to a set of data ingestion stages of a data ingestion of the DICOM data from a radiology device to a database.
FIG. 5 is a flowchart of an example process for determining a data quality of DICOM data.
FIG. 6 is a diagram of example validation rules for determining a data quality of DICOM.
FIG. 7 is a diagram of an example user interface for displaying a data quality of DICOM data associated with a radiology device.
FIG. 8 is a diagram of an example user interface for displaying respective data qualities of DICOM data corresponding to validation rules.
FIG. 9 is a flowchart of an example process for determining a root cause of a data quality not satisfying a threshold.
FIG. 10 is a flowchart of an example process for performing an action to improve the data quality of DICOM data.
FIG. 11 is a diagram of an example process for training an artificial intelligence (AI) model.

### DETAILED DESCRIPTION

Embodiments of the present disclosure will now be described, by way of example, with reference to the figures.

As addressed above, the task of assessing data quality of a large number of radiology devices might prove impossible, inefficient, error-prone, or the like. This problem is further exacerbated based on the radiology devices generating DICOM data having substantial variability depending on the imaging modality, manufacturer, model, etc., of the respective radiology devices.

Some embodiments of the present disclosure provide a data quality platform that may receive information related to a data ingestion of DICOM data from a radiology device to a database, and determine, using a set of data validation rules corresponding to a set of data ingestion stages of the data ingestion of the DICOM data from the radiology device to the database, respective data qualities of the DICOM data at the set of data ingestion stages of the data ingestion. Further, the data quality platform may be configured to generate a user interface that displays information related to the respective data qualities of the DICOM data at the set of data ingestion stages of the data ingestion.

Some embodiments of the present disclosure provide a technical improvement in the technical field of radiology and a technical improvement to devices associated with the acquisition, ingestion, and usage of DICOM data of radiology devices by determining data quality of DICOM data at a set of data ingestion stages of a data ingestion of the DICOM data. In the event that the data quality does not satisfy a threshold, some embodiments of the present disclosure provide for the identification of a root cause of the data quality not satisfying the threshold and/or the action of improving the data quality of the DICOM data.

In this way, some embodiments of the present disclosure improve the safety, efficacy, and reliability of DICOM data by ensuring adequate data quality, eliminate, or reduce, the need for human intervention in assessing data quality, accelerate the serviceability of radiology devices, improve transparency in data ingestion systems, improve the identification of a root cause of issues using data lineage, automatically retain DICOM data in the event that the data quality does not satisfy a threshold, automatically identify issues associated with data ingestion, decrease the time needed to connect, integrate, and validate a radiology device, and improve the usage of the AI models trained using the DICOM data.

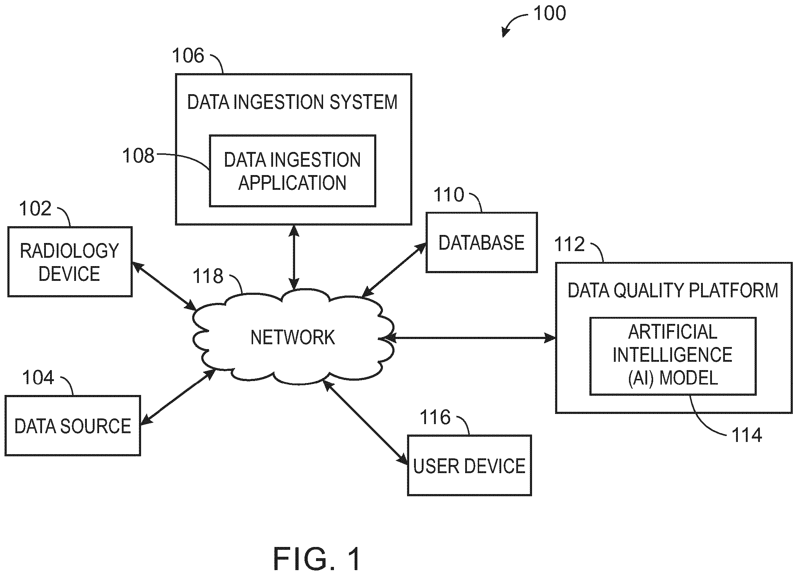

FIG. 1 is a diagram of an example system 100 for determining a data quality of DICOM data. As shown in FIG. 1, the system 100 may include a radiology device 102, a data source 104, a data ingestion system 106, a data ingestion application 108, a database 110, a data quality platform 112, an AI model 114, a user device 116, and a network 118.

The radiology device 102 may be configured to generate DICOM data using an imaging modality. For example, the radiology device 102 may be an ultrasound device, an X-ray device, an electrocardiogram (ECG) device, a computed tomography (CT) device, a magnetic resonance imaging (MRI) device, a positron emission tomography (PET) device, or the like. The radiology device 102 may be associated with a particular manufacturer identified by a manufacturer identifier, may be associated with a particular model identified by a model identifier, or the like. The DICOM data may include a header and a data set. The data set may include data elements as defined by the DICOM standard. For example, the data elements may include a patient identifier, a name, a sex, an age, a type of procedure, a device type, a procedure description, a procedure code, pixel data of a medical image generated by the radiology device 102, or the like. The data elements may be associated with corresponding tags.

The data source 104 may be configured to provide information associated with a patient, information associated with a radiology device 102, information associated with the data ingestion system 106, or the like. For example, the data source 104 may be a server, a database, or the like.

The data ingestion system 106 may be configured to perform data ingestion of DICOM data from the radiology device 102 to the database 110. For example, the data ingestion system 106 may be a data hub, a data lake, a data warehouse, a database, or the like. The data ingestion application 108 may be configured to process the DICOM data in association with the data ingestion from the radiology device 102 to the database 110. For example, the data ingestion application 108 may be a data extraction application that is configured to perform data extraction using the DICOM data. As another example, the data ingestion application 108 may be a data enhancement application configured to perform data enhancement using the DICOM data. As another example, the data ingestion application 108 may be a data enrichment application configured to perform data enrichment using the DICOM data. The data ingestion may include a set of data ingestion stages. For example, the data ingestion may include a data extraction stage in which the data ingestion application 108 performs data extraction using the DICOM data. As another example, the data ingestion may include a data enhancement stage in which the data ingestion application 108 performs data enhancement using the DICOM data. As another example, the data ingestion may include a data enrichment stage in which the data ingestion application 108 performs data enrichment using the DICOM data. It should be understood that the data ingestion may include any number of stages. Further, it should be understood that multiple data ingestion applications 108 may perform respective data enrichments of the DICOM data. According to an embodiment, the data ingestion system 106 may store information identifying the DICOM data at each respective data ingestion stage of the data ingestion stages. The DICOM data may vary at each respective data ingestion stage. For example, various data elements of the DICOM data may have adjusted values, added values, modified values, removed values, enhanced values, etc., depending on the data ingestion applications 108 that process the DICOM data in association with the data ingestion.

The database 110 may be configured to store the DICOM data. For example, the database 110 may be a hierarchical database, a network database, a relational database, or the like. The database 110 may store information related to the data ingestion of the DICOM data. For example, the database 110 may store the DICOM data, a DICOM data identifier that identifies the DICOM data, a radiology device identifier that identifies the radiology device 102 that generated the DICOM data, a manufacturer identifier that identifies a manufacturer of the radiology device 102, a model identifier that identifies a model of the radiology device 102, a site identifier that identifies a site associated with the radiology device 102, a time identifier that identifies a time associated with the DICOM data (e.g., a time of data ingestion, a time of data generation, or the like), or the like. According to an embodiment, the database 110 may store information identifying the DICOM data at each respective stage of the data ingestion stages.

The data quality platform 112 may be configured to receive information related to the data ingestion of DICOM data from the radiology device 102 to the database 110. Further, the data quality platform 112 may be configured to determine, using a set of data validation rules corresponding to a set of data ingestion stages of the data ingestion of the DICOM data from the radiology device 102 to the database 110, respective data qualities of the DICOM data at the set of data ingestion stages of the data ingestion. Further, the data quality platform 112 may be configured to generate a user interface that displays information related to the respective data qualities of the DICOM data at the set of data ingestion stages of the data ingestion.

The AI model 114 may be configured to receive DICOM data as an input, determine a data quality of the DICOM data, and provide the data quality of the DICOM data as an output. For example, the AI model 114 may be a deep neural network (DNN), a convolutional neural networks (CNN), a fully convolutional network (FCN), a recurrent neural network (RCN), a Bayesian network, a graphical probabilistic model, a K-nearest neighbor classifier, a decision forests, a maximum margin method, or the like.

The user device 116 may be configured to display information related to the respective data qualities of the DICOM data at the set of data ingestion stages of the data ingestion. For example, the user device 116 may be a smartphone, a laptop computer, a desktop computer, a wearable device, or the like.

The network 118 may be configured to permit communication between the radiology device 102, the data source 104, the data ingestion system 106, the database 110, the data quality platform 112, and/or the user device 116. For example, the network 118 may be a cellular network (e.g., a fifth generation (5G) network, a long-term evolution (LTE) network, a third generation (3G) network, a code division multiple access (CDMA) network, etc.), a public land mobile network (PLMN), a local area network (LAN), a wide area network (WAN), a metropolitan area network (MAN), a telephone network (e.g., the Public Switched Telephone Network (PSTN)), a private network, an ad hoc network, an intranet, the Internet, a fiber optic-based network, or the like, and/or a combination of these or other types of networks.

The number and arrangement of the devices of the system 100 shown in FIG. 1 are provided as an example. In practice, the system 100 may include additional devices, fewer devices, different devices, or differently arranged devices than those shown in FIG. 1. Additionally, or alternatively, a set of devices (e.g., one or more devices) of the system 100 may perform one or more functions described as being performed by another set of devices of the system 100.

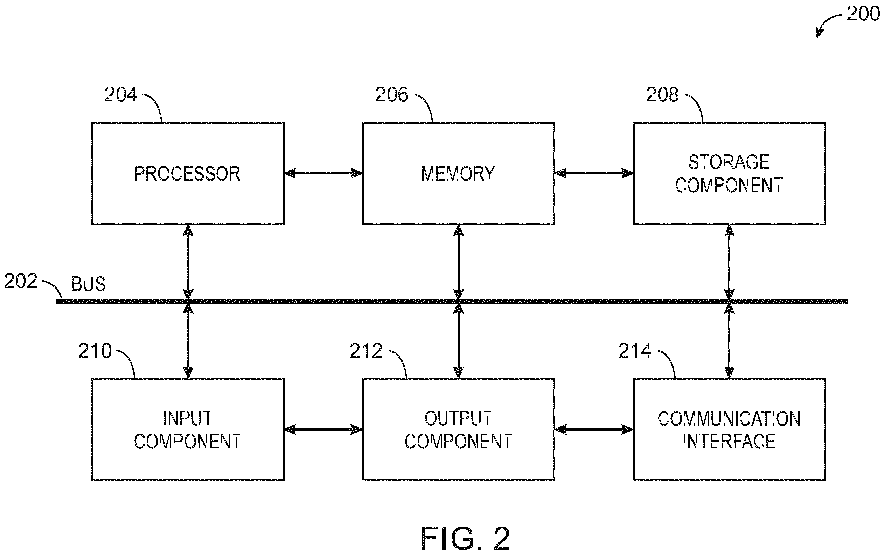

FIG. 2 is a diagram of example components of a device 200 of FIG. 1. The device 200 may correspond to the radiology device 102, the data source 104, the data ingestion system 106, the database 110, the data quality platform 112, and/or the user device 116. As shown in FIG. 2, the device 200 may include a bus 202, a processor 204, a memory 206, a storage component 208, an input component 210, an output component 212, and a communication interface 214.

The bus 202 includes a component that permits communication among the components of the device 200. The processor 204 may be implemented in hardware, firmware, or a combination of hardware and software. The processor 204 may be a central processing unit (CPU), a graphics processing unit (GPU), an accelerated processing unit (APU), a microprocessor, a microcontroller, a digital signal processor (DSP), a field-programmable gate array (FPGA), an application-specific integrated circuit (ASIC), or another type of processing component.

The processor 204 may include one or more processors capable of being programmed to perform a function. The memory 206 may include a random access memory (RAM), a read only memory (ROM), and/or another type of dynamic or static storage device (e.g., a flash memory, a magnetic memory, and/or an optical memory) that stores information and/or instructions for use by the processor 204.

The storage component 208 may store information and/or software related to the operation and use of the device 200. For example, the storage component 208 may include a hard disk (e.g., a magnetic disk, an optical disk, a magneto-optic disk, and/or a solid state disk), a compact disc (CD), a digital versatile disc (DVD), a floppy disk, a cartridge, a magnetic tape, and/or another type of non-transitory computer-readable medium, along with a corresponding drive.

The input component 210 may include a component that permits the device 200 to receive information, such as via user input (e.g., a touch screen display, a keyboard, a keypad, a mouse, a button, a switch, a camera, and/or a microphone). Additionally, or alternatively, the input component 210 may include a sensor for sensing information (e.g., a global positioning system (GPS) component, an accelerometer, a gyroscope, and/or an actuator). The output component 212 may include a component that provides output information from the device 200 (e.g., a display, a speaker for outputting sound at the output sound level, and/or one or more light-emitting diodes (LEDs)).

The communication interface 214 may include a transceiver-like component (e.g., a transceiver and/or a separate receiver and transmitter) that enables the device 200 to communicate with other devices, such as via a wired connection, a wireless connection, or a combination of wired and wireless connections. The communication interface 214 may permit the device 200 to receive information from another device and/or provide information to another device. For example, the communication interface 214 may include an Ethernet interface, an optical interface, a coaxial interface, an infrared interface, a radio frequency (RF) interface, a universal serial bus (USB) interface, a Wi-Fi interface, a cellular network interface, or the like.

The device 200 may perform one or more processes described herein. The device 200 may perform these processes based on the processor 204 executing software instructions stored by a non-transitory computer-readable medium, such as the memory 206 and/or the storage component 208. A computer-readable medium may be defined herein as a non-transitory memory device. A memory device may include memory space within a single physical storage device or memory space spread across multiple physical storage devices.

The software instructions may be read into the memory 206 and/or the storage component 208 from another computer-readable medium or from another device via the communication interface 214. When executed, the software instructions stored in the memory 206 and/or the storage component 208 may cause the processor 204 to perform one or more processes described herein. Additionally, or alternatively, hardwired circuitry may be used in place of or in combination with software instructions to perform one or more processes described herein. Thus, implementations described herein are not limited to any specific combination of hardware circuitry and software.

The number and arrangement of the components shown in FIG. 2 are provided as an example. In practice, the device 200 may include additional components, fewer components, different components, or differently arranged components than those shown in FIG. 2. Additionally, or alternatively, a set of components (e.g., one or more components) of the device 200 may perform one or more functions described as being performed by another set of components of the device 200.

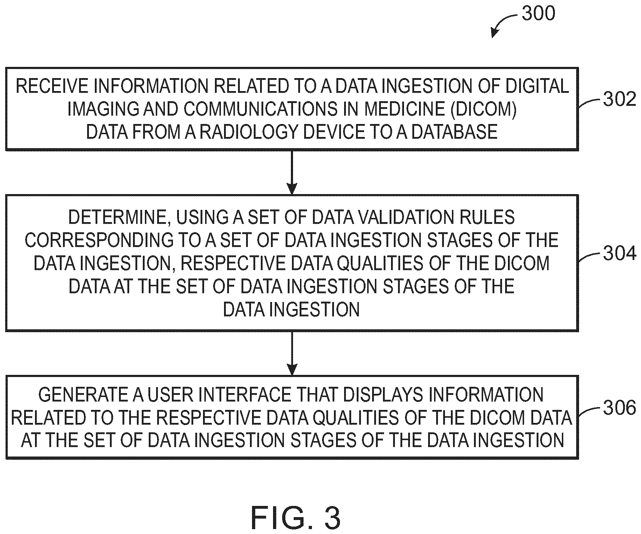

FIG. 3 is a diagram of a flowchart of an example process 300 for determining a data quality of DICOM data using a set of validation rules corresponding to a set of data ingestion stages of a data ingestion of the DICOM data from a radiology device to a database. The data quality platform 112 may be configured to perform the operations of the process 300.

As shown in FIG. 3, the process 300 may include receiving information related to a data ingestion of DICOM data from a radiology device to a database (operation 302). According to an embodiment, the information related to the data ingestion may include the DICOM data. Additionally, or alternatively, the information related to the data ingestion may include the DICOM data at each respective data ingestion stage of the data ingestion stages. Additionally, or alternatively, the information related to the data ingestion may include information identifying data ingestion rules associated with the data ingestion performed by the data ingestion system 106. Additionally, or alternatively, the information related to the data ingestion may include information identifying the set of data ingestion stages of the data ingestion. Additionally, or alternatively, the information related to the data ingestion may include information identifying a set of data ingestion applications 108 that processed the DICOM data. Additionally, or alternatively, the information related to the data ingestion may include information identifying respective rules of the set of data ingestion applications 108 that processed the DICOM data.

According to an embodiment, the data quality platform 112 may receive the information related to the data ingestion of the DICOM data at a predetermined interval. For example, the data quality platform 112 may receive information related to data ingestion of DICOM data every ten minutes, every hour, every twenty four hours, or the like. Additionally, or alternatively, the data quality platform 112 may receive information related to data ingestion of DICOM data in substantially real-time. Herein, "substantially real-time" may refer to receiving the DICOM data within a threshold time (e.g., ten seconds, one minute, etc.) of a reference time (e.g., the radiology device 102 generating the DICOM data, the data ingestion system 106 performing the data ingestion, the database 110 storing the DICOM data, or the like). Additionally, or alternatively, the data quality platform 112 may receive the information related to the data ingestion of the DICOM data based on a request from the user device 116.

As further shown in FIG. 3, the process 300 may include determining, using a set of validation rules corresponding to a set of data ingestion stages of the data ingestion, respective data qualities of the DICOM data at the set of data ingestion stages of the data ingestion (operation 304).

The data quality may be a representation of an actual state of the DICOM data as compared to a predetermined state. According to an embodiment, the data quality may be represented as a data quality metric. For example, the data quality metric may be an accuracy of the DICOM data, a completeness of the DICOM data, a comprehensiveness of the DICOM data, a consistency of the DICOM data, a timeliness of the DICOM data, a uniqueness of the DICOM data, a validity of the DICOM data, or the like.

The data quality platform 112 may determine a data quality of the DICOM data at a data ingestion stage of the data ingestion using a data validation rule that corresponds to the data ingestion stage. The data ingestion may include a set of data ingestion stages (e.g., a single data ingestion stage, ten data ingestion stages, one hundred data ingestion stages, or the like). The data quality platform 112 may determine respective data qualities of the DICOM data at the set of data ingestion stages. For example, the data quality platform 112 may determine a data quality of the DICOM data at one or more data ingestion stages of the data ingestion stages. The data quality platform 112 may determine a data quality of the DICOM data at a particular data ingestion stage using a particular data validation rule that corresponds to the particular data ingestion stage. The data quality platform 112 may determine the particular data validation rule to be applied at the particular data ingestion stage based on information related to the data ingestion.

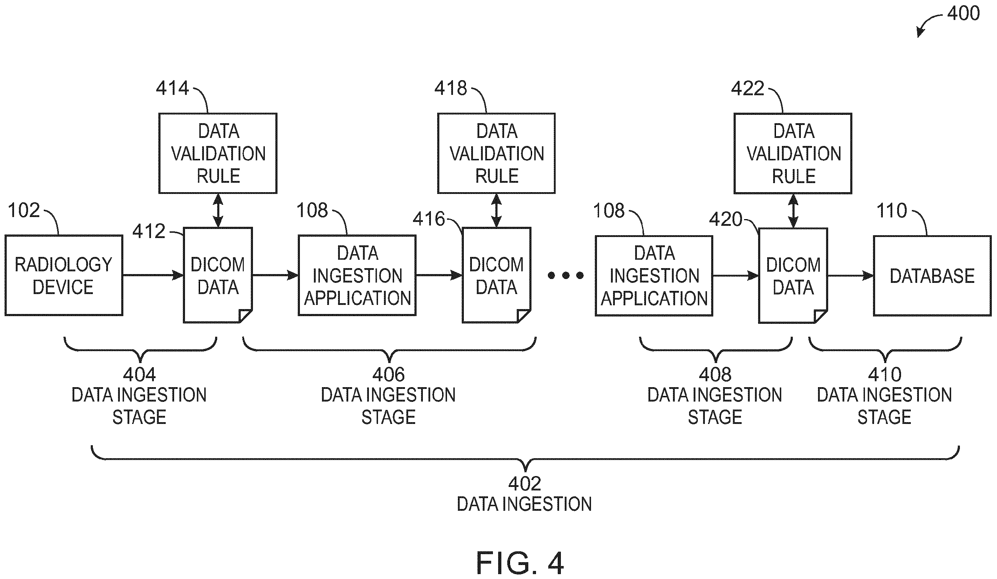

FIG. 4 is a diagram of an example process 400 for determining a data quality of DICOM data using a set of validation rules corresponding to a set of data ingestion stages of a data ingestion of the DICOM data from a radiology device to a database. As shown in FIG. 4, the data ingestion 402 may include a set of data ingestion stages, such as the data ingestion stage 404, the data ingestion stage 406, the data ingestion stage 408, and the data ingestion stage 410. At the data ingestion stage 404, the radiology device 102 may provide the DICOM data 412 to the data ingestion system 106. The data quality platform 112 may determine a data quality of the DICOM data 412 using the data validation rule 414 that corresponds to the data ingestion stage 404. At the data ingestion stage 406, the data ingestion system 106 may input the DICOM data 412 into the data ingestion application 108, and receive the DICOM data 416 output by the data ingestion application 108. The data quality platform 112 may determine a data quality of the DICOM data 416 using the data validation rule 418 that corresponds to the data ingestion stage 406. At the data ingestion stage 408, the data ingestion system 106 may receive the DICOM data 420 output by the data ingestion application 108. The data quality platform 112 may determine a data quality of the DICOM data of the DICOM data 420 using the data validation rule 422 that corresponds to the data ingestion stage 408. At the data ingestion stage 410, the data ingestion system 106 may provide the DICOM data 420 to the database 110. The data quality platform 112 may determine a data quality of the DICOM data 420 using the data validation rule 422 that corresponds to the data ingestion stage 410.

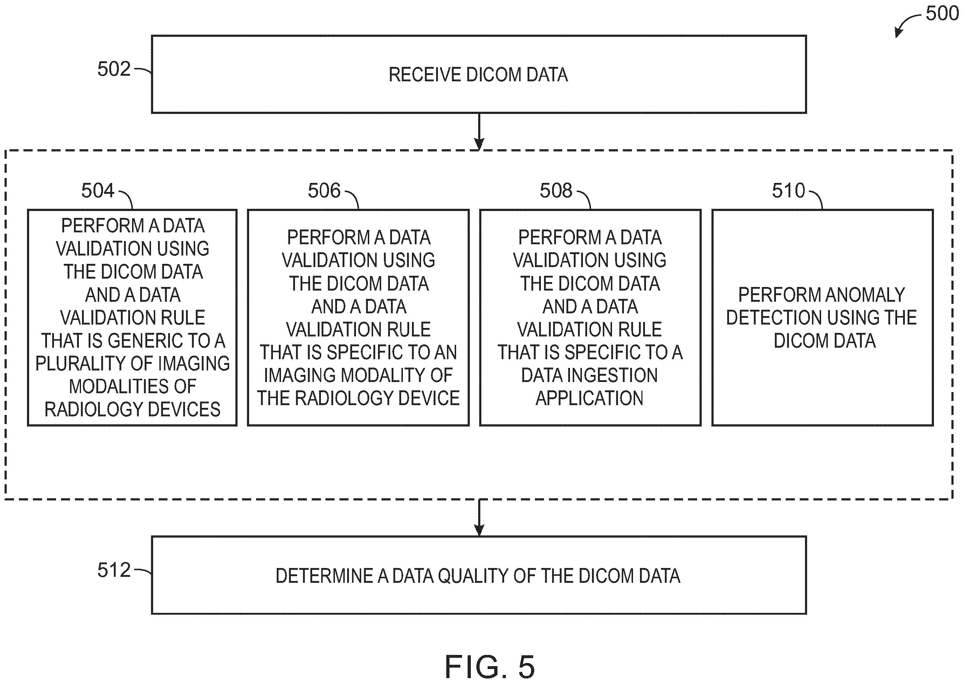

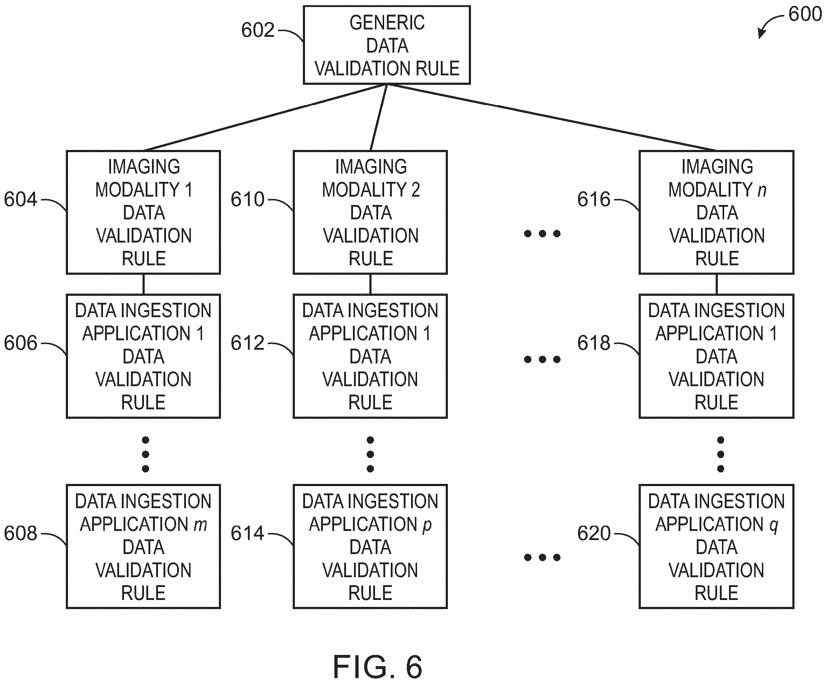

The data quality platform 112 may receive the DICOM data, perform a data validation using a data validation rule, and determine a data quality of the DICOM data based on performing the data validation. FIG. 5 is a flowchart of an example process 500 for determining a data quality of DICOM data. FIG. 6 is a diagram of example validation rules 600 for determining a data quality of DICOM.

As shown in FIG. 5, the process 500 may include receiving DICOM data (operation 502). For example, the data quality platform 112 may receive DICOM data corresponding to a particular data ingestion stage from the database 110 or the data ingestion system 106.

As further shown in FIG. 5, the process 500 may include performing a data validation using the DICOM data and a data validation rule that is generic to a plurality of imaging modalities of radiology devices (operation 504). The data validation rule may be generic to a plurality of imaging modalities of radiology devices 102. For example, the data validation rule may correspond to whether a particular data element (e.g., a study identifier, a patient identifier, a patient sex, etc.) has a value in the DICOM data. As shown in FIG. 6, the data quality platform 112 may determine a data quality of the DICOM data using a data validation rule 602 that is generic to a plurality of imaging modalities of radiology devices 102. For instance, the data quality platform 112 may use the data validation rule 602 when determining a data quality of DICOM data of a first radiology device 102 associated with a first imaging modality, and may also use the data validation rule 602 when determining a data quality of DICOM data of a second radiology device 102 associated with a second imaging modality that is different than the first imaging modality.

As further shown in FIG. 5, the process 500 may include performing a data validation using the DICOM data and a data validation rule that is specific to an imaging modality of the radiology device 102 (operation 506). The data validation rule may be specific to an imaging modality of the radiology device 102 that generated the DICOM data. For example, the data validation rule may correspond to a parameter or a configuration of the radiology device 102. As an example, if the data validation rule corresponds to an ultrasound imaging modality, then the data quality platform 112 may determine whether a duration of the ultrasound imaging is within a particular time range defined by a minimum time and maximum time. If the data quality platform 112 determines that the duration is not within the particular time range, then the data quality platform 112 may not validate the DICOM data. As another example, if the data validation rule corresponds to a CT imaging modality, then the data quality platform 112 may determine whether an acquisition parameter (e.g., a current value, a voltage value, or the like) is within a range defined by a minimum value and a maximum value. If the data quality platform 112 determines that the acquisition parameter is not within the particular time range, then the data quality platform 112 may not validate the DICOM data. As shown in FIG. 6, the data quality platform 112 may determine a data quality using a data validation rule 604 that is specific to a first imaging modality, determine a data quality using a data validation rule 610 that is specific to a second imaging modality, and determine a data quality using an *n*-th data validation rule 616 that is specific to an *n*-th imaging modality. For instance, the data quality platform 112 may use the data validation rule 604 only for radiology devices 102 of the first imaging modality, may use the data validation rule 610 only for radiology devices 102 of the second imaging modality, etc.

As further shown in FIG. 5, the process 500 may include performing a data validation using the DICOM data and a data validation rule that is specific to a data ingestion application (operation 508). For example, the data validation rule may correspond to a particular data ingestion application 108. As an example, the data quality platform 112 may determine whether a score of an output image output by a data ingestion application 108 configured to perform data enrichment is within a range defined by a minimum value and a maximum value. As shown in FIG. 6, the data quality platform 112 may determine the data quality using a data validation rule 606 that is specific to a first data ingestion application 108 associated with a first imaging modality, determine a data quality using a data validation rule 608 that is specific to an *m*-th data ingestion application 108 associated with the first imaging modality, determine a data quality using a data validation rule 612 that is specific to a first data ingestion application 108 associated with a second imaging modality, determine a data quality using a data validation rule 614 that is specific to a p-th data ingestion application 108 associated with the second imaging modality, determine a data quality using a data validation rule 618 associated with a first data ingestion application 108 associated with an *n*-th imaging modality, and determine a data quality using a data validation rule 620 associated with a *q*-th data ingestion application 108 associated with the *n*-th imaging modality. For instance, the data quality platform 112 may use the data validation rule 606 only in association with the first data ingestion application 108 associated with the first imaging modality. According to an embodiment, the data ingestion application 108 may generate a new data element for the DICOM data that are pertinent to medical data usage. The data quality platform 112 may determine whether a range of the new data element is within a particular range.

As further shown in FIG. 5, the process 500 may include performing anomaly detection using the DICOM data (operation 510). For example, the data quality platform 112 may perform anomaly detection using an anomaly detection technique, such as a supervised anomaly detection technique, a semi-supervised anomaly detection technique, an unsupervised anomaly detection technique, a statistical technique, a density-based technique, a subspace technique, a correlation-based technique, a neural network, a Bayesian network, or the like. By performing anomaly detection, the data quality platform 112 may determine whether the DICOM data deviates from previous DICOM data, determine whether the DICOM data includes values that rarely occur, determine whether the DICOM data is inconsistent with previous DICOM data, or the like. As an example, the data quality platform 112 may determine whether an exam duration repartition is consistent across time. If the distribution of values of a certain data element of the DICOM data for a particular radiology device 102 deviates by a substantial amount, then the data quality platform 112 may detect an anomaly.

According to an embodiment, the data quality platform 112 may determine a data quality using the AI model 114. For example, the data quality platform 112 may input the DICOM data into the AI model 114, and determine a data quality of the DICOM data based on an output of the AI model 114.

As further shown in FIG. 5, the process 500 may include determining a data quality of the DICOM data (operation 512). For example, the data quality platform 112 may determine a data quality of the DICOM data based on performing operation 504, operation 506, operation 508, or operation 510. According to an embodiment, the data quality of the DICOM data may correspond to the collective data set. For example, the data quality platform 112 may determine respective data qualities of data elements of the data set, and determine a collective data quality of the data set based on the respective data qualities. Additionally, or alternatively, the data quality may correspond to a particular data element of the dataset. For example, the data quality platform 112 may determine a data quality of a particular data element of the data set, and determine the particular data quality as the data quality.

In this way, the data quality platform 112 may determine respective data qualities of one or more data elements of the DICOM data at one or more data ingestion stages of the set of data ingestion stages of the data ingestion from the radiology device 102 to the database 110. Accordingly, in this way, the embodiments herein improve the safety, efficacy, and reliability of DICOM data by ensuring adequate data quality, eliminate, or reduce, the need for human intervention in assessing data quality, accelerate the serviceability of radiology devices, improve transparency in data ingestion systems, improve the identification of a root cause of issues using data lineage, automatically retain DICOM data in the event that the data quality does not satisfy a threshold, automatically identify issues associated with data ingestion, decrease the time needed to connect, integrate, and validate a radiology device, and improve the usage of the AI models trained using the DICOM data.

As further shown in FIG. 3, the process 300 may include generating a user interface that displays information related to the respective data qualities of the DICOM data at the set of data ingestion stages of the data ingestion (operation 306). The data quality platform 112 may generate a user interface, and provide the user interface to the user device 116 to permit the user device 116 to display information related to the respective data qualities of the DICOM data at the set of data ingestion stages of the data ingestion from the radiology device 102 to the database 110.

According to an embodiment, the information related to the data quality may be a status of the data quality. For example, the status may be "normal," "pass," "accepted," "valid," "high," "medium," "low," "fail," "warning," or the like. Additionally, or alternatively, the information related to the data quality may be a discrete value for the data quality. For example, the discrete value may be "100%," "90%," "7," "0.5," or the like. Additionally, or alternatively, the information related to the data quality may be an indicator of a particular data ingestion stage associated with the DICOM data. Additionally, or alternatively, the information related to the data quality may be the DICOM data. Additionally, or alternatively, the information related to the data quality may be a DICOM data identifier that identifies the DICOM data. Additionally, or alternatively, the information related to the data quality may be a tag of a data element that contributed to the data quality not satisfying a threshold. Additionally, or alternatively, the information related to the data quality may be a radiology device identifier that identifies the radiology device 102 that generated the DICOM data. Additionally, or alternatively, the information related to the data quality may be a manufacturer identifier that identifies a manufacturer of the radiology device 102. Additionally, or alternatively, the information related to the data quality may be a model identifier that identifies a model of the radiology device 102. Additionally, or alternatively, the information related to the data quality may be a site identifier that identifies a site associated with the radiology device 102. Additionally, or alternatively, the information related to the data quality may be a time identifier that identifies a time associated with the DICOM data (e.g., a time of data ingestion, a time of data generation, or the like), or the like. Additionally, or alternatively, the information related to the data quality may be an identifier of a data validation rule that was applied to the DICOM data. Additionally, or alternatively, the information related to the data quality may be an identifier of a data ingestion application 108 that processed the DICOM data.

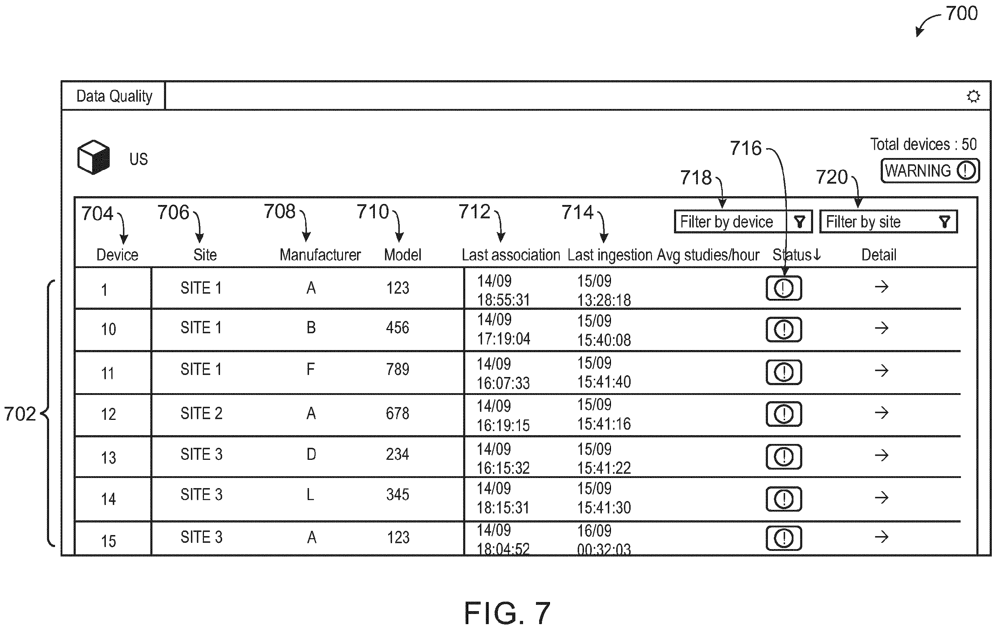

FIG. 7 is a diagram of an example user interface 700 for displaying a data quality of DICOM data associated with a radiology device. As shown in FIG. 7, the user interface 700 may display data 702 corresponding to a set of radiology devices 102. The data 702 may include a device identifier 704 of the radiology device 102, a site identifier 706 that identifies a site associated with the radiology device 102, a manufacturer identifier 708 that identifies a manufacturer of the radiology device 102, a model identifier 710 that identifies a model of the radiology device 102, a last association identifier 712 that identifies a last association of the radiology device 102, a last data ingestion identifier 714 that identifies a last data ingestion of the radiology device 102, and a status identifier 716 of the data quality of the DICOM data of the radiology device 102. The user interface 700 may include a field 718 that permits an operator to filter the data 702 by a particular radiology device 102. The user interface 700 may include a field 718 that permits an operator to filter the data 702 by a particular site.

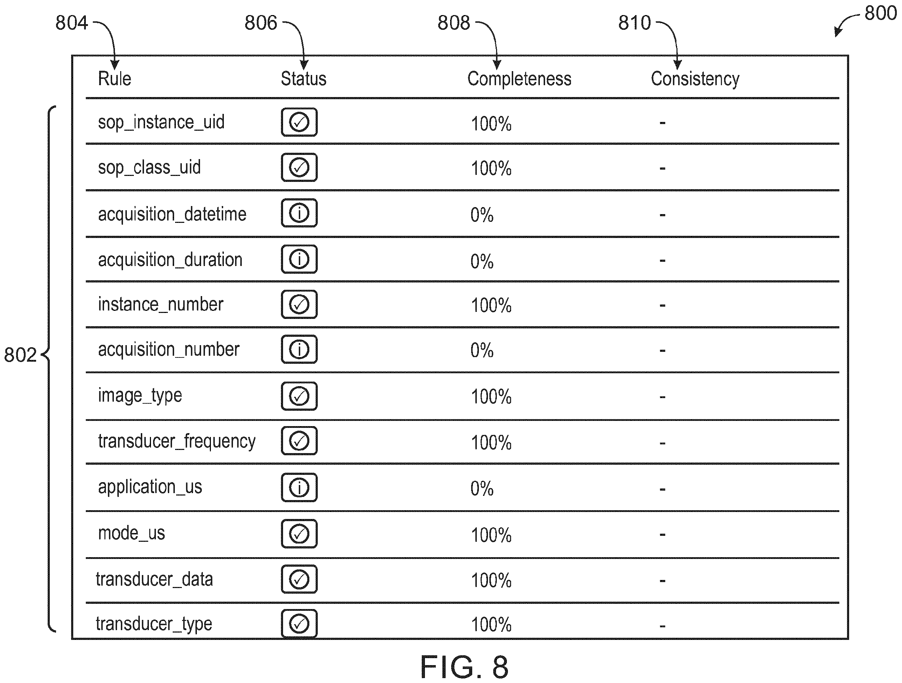

FIG. 8 is a diagram of an example user interface 800 for displaying respective data qualities of DICOM data corresponding to validation rules. As shown in FIG. 8, the user interface 800 may display data 802 corresponding to respective data qualities of DICOM data of a radiology device 102 corresponding to validation rules. As shown, the user interface 800 may display data validation rules 804, statuses 806 of the DICOM data with respect to the data validation rules, completeness metrics 808 of the DICOM data with respect to the data validation rules, consistency metrics 810 of the DICOM data with respect to the data validation rules, or the like.

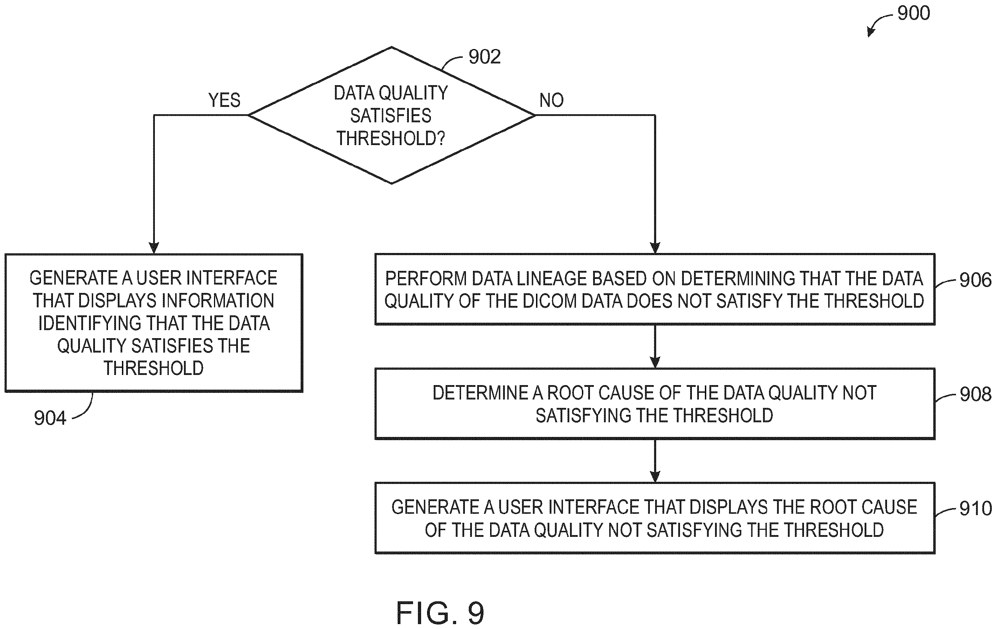

FIG. 9 is a flowchart of an example process 900 for determining a root cause of a data quality not satisfying a threshold. The data quality platform 112 may be configured to perform the operations of the process 900.

As shown in FIG. 9, the process 900 may include determining whether a data quality of the DICOM data satisfies a threshold (operation 902). According to an embodiment, the data quality platform 112 may compare the respective data quality of the DICOM data at the one or more data ingestion stages of the data ingestion to a respective threshold, and determine whether the respective data quality satisfies the respective threshold. For example, the data quality platform 112 may compare a value of a data quality of the DICOM data to a threshold value, and determine whether the value of the data quality satisfies the threshold value. As a particular example, the data quality platform 112 may compare a value of "90%" to a threshold value of "70%." In this case, "satisfies the threshold value" may refer to the value of the data quality being greater than or greater than or equal to the threshold value. As another example, the data quality platform 112 may compare a status of the data quality to a threshold status. As a particular example, the data quality platform 112 may compare a status of "high" to a threshold status of "medium." In this case, "satisfies the threshold value" may refer to the status of the data quality being greater than or greater than or equal to the threshold status.

As further shown in FIG. 9, if the data quality satisfies the threshold (operation 902 - YES), then the process 900 may include generating a user interface that displays information identifying that the data quality satisfies the threshold (operation 904). For example, the data quality platform 112 may generate a user interface that displays a status of the DICOM data that identifies that the DICOM data satisfies the threshold. Additionally, the data quality platform 112 may display respective statuses of the DICOM data with respect to various data validation rules that indicate that the DICOM data satisfies the threshold.

As further shown in FIG. 9, if the data quality does not satisfy the threshold (operation 902 - NO), then the process 900 may include performing data lineage based on determining that the data quality of the DICOM data does not satisfy the threshold (operation 906), and determining a root cause of the data quality not satisfying the threshold (operation 908).

According to an embodiment, the data quality platform 112 may determine a data ingestion stage that is associated with DICOM data having the data quality that does not satisfy the threshold. According to an embodiment, the data quality platform 112 may perform backward data lineage to determine a root cause of the DICOM data not satisfying the threshold. As an example, the data quality platform 112 may perform backward data lineage to determine that a root cause of the DICOM data not satisfying the threshold is an incorrect configuration of the radiology device 102. As another example, the data quality platform 112 may perform backward data lineage to determine that a root cause of the DICOM data not satisfying the threshold is an incorrect configuration of a data ingestion application 108 that processed the DICOM data. According to an embodiment, the data quality platform 112 may perform forward data lineage to determine data ingestion applications 108 that might be affected by the DICOM data not satisfying the threshold. The data quality platform 112 may prevent the data ingestion applications 108 from accessing the DICOM data having the data quality that does not satisfy the threshold. The data quality platform 112 may determine DICOM data associated with downstream data ingestion stages of the data ingestion stage that is associated with the DICOM data having the data quality that does not satisfy the threshold, and perform an action using the DICOM data (e.g., flag the DICOM data as suspicious, determine a data quality of the DICOM data, or the like).

According to an embodiment, the data quality platform 112 may generate a graph structure based on the information related to the data ingestion. For example, the data quality platform 112 may generate the graph structure based on information associated with the data ingestion system 106, information associated with the data ingestion applications 108, or the like. The graph structure may include a set of nodes corresponding to the DICOM data at the various data ingestion stages of the data ingestion, and edges that connect the set of nodes and that identify relationships between the set of nodes. For instance, the edges may identify the data ingestion applications 108 that process the DICOM data at the various data ingestion stages. The data quality platform 112 may use the graph structure to perform the data lineage. According to another embodiment, the data quality platform 112 may perform log parsing to perform the data lineage.

As further shown in FIG. 9, the process 900 may include generating a user interface that displays the root cause of the data quality not satisfying the threshold (operation 910). For example, the data quality platform 112 may generate a user interface that displays the root cause, and provide the user interface to the user device 116 for display. Additionally, the data quality platform 112 may generate the user interface to include information identifying potential actions to perform to improve the data quality of the DICOM data. In this way, an operator of the user device 116 may identify the root cause, and perform an action to improve the data quality of the DICOM data.

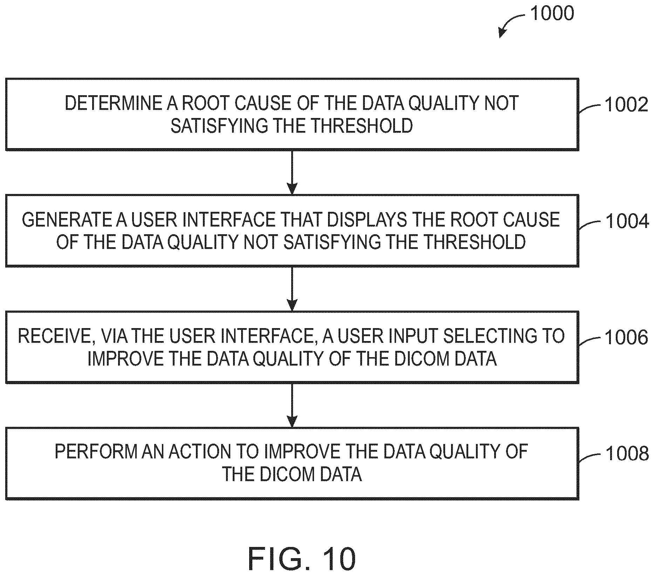

FIG. 10 is a flowchart of an example process 1000 for performing an action to improve the data quality of DICOM data. The data quality platform 112 may be configured to perform the operations of the process 1000.

As shown in FIG. 10, the process 1000 may include determining a root cause of the data quality not satisfying the threshold (operation 1002), and generating a user interface that displays the root cause of the data quality not satisfying the threshold (operation 1004). For example, the data quality platform 112 may determine a root cause of the data quality not satisfying the threshold in a similar manner as described above in connection with operation 902 of FIG. 9, and may generate a user interface that displays the root cause of the data quality not satisfying the threshold in a similar manner as described above in connection with operation 908 of FIG. 9.

As further shown in FIG. 10, the process 1000 may include receiving, via the user interface, a user input selecting to improve the data quality of the DICOM data (operation 1006), and performing an action to improve the data quality of the DICOM data (operation 1008).

The data quality platform 112 may generate the user interface to display information identifying the root cause of the data quality not satisfying the threshold, and to display information that permits an operator to select to improve the data quality of the DICOM data. For example, if the root cause of the data quality not satisfying the threshold is an incorrect configuration of the radiology device 102, then the user interface may display information that permits an operator to select to adjust the configuration of the radiology device 102 and/or to perform additional imaging of the patient using the adjusted configuration of the radiology device. As another example, if the root cause of the data quality not satisfying the threshold is an incorrect configuration of a data ingestion application 108, then the user interface may display information that permits an operator to select to adjust the configuration of the data ingestion application 108 and/or to perform an updated data enhancement using the data ingestion application 108 with the adjusted configuration. As another example, if the root cause of the data quality not satisfying the threshold is an omission of a value for a data element, then the data quality platform 112 may display information that permits the operator to select to input a value for the data element. As another example, if the root cause of the data quality not satisfying the threshold is an incorrect format of a value for a data element, then the data quality platform 112 may display information that permits the operator to select to reformat the value for the data element. Based on a user selection, the data quality platform 112 may perform an action to improve the data quality of the DICOM data. For example, the data quality platform 112 may adjust a configuration (e.g., an acquisition parameter, a setting, or the like) of a radiology device 102. As another example, the data quality platform 112 may cause a radiology device 102 to perform an additional scan of a patient. As another example, the data quality platform 102 may prevent a radiology device 102 from performing an additional scan of a patient. As another example, the data quality platform 112 may adjust a configuration of a data ingestion application 108. As another example, the data quality platform 112 may prevent a data ingestion application 108 from processing DICOM data during data ingestion. As another example, the data quality platform 112 may input a value for a data element. As another example, the data quality platform 112 may reformat a value for a data element. As another example, the data quality platform 112 may transmit a notification to another device. As another example, the data quality platform 112 may retain the DICOM data, such as by preventing the DICOM data from being displayed, from being accessed by another device, from being transmitted to another device, or the like.

In this way, the embodiments herein improve the safety, efficacy, and reliability of DICOM data by ensuring adequate data quality, eliminate, or reduce, the need for human intervention in assessing data quality, accelerate the serviceability of radiology devices, improve transparency in data ingestion systems, improve the identification of a root cause of issues using data lineage, automatically retain DICOM data in the event that the data quality does not satisfy a threshold, automatically identify issues associated with data ingestion, decrease the time needed to connect, integrate, and validate a radiology device, and improve the usage of the AI models trained using the DICOM data.

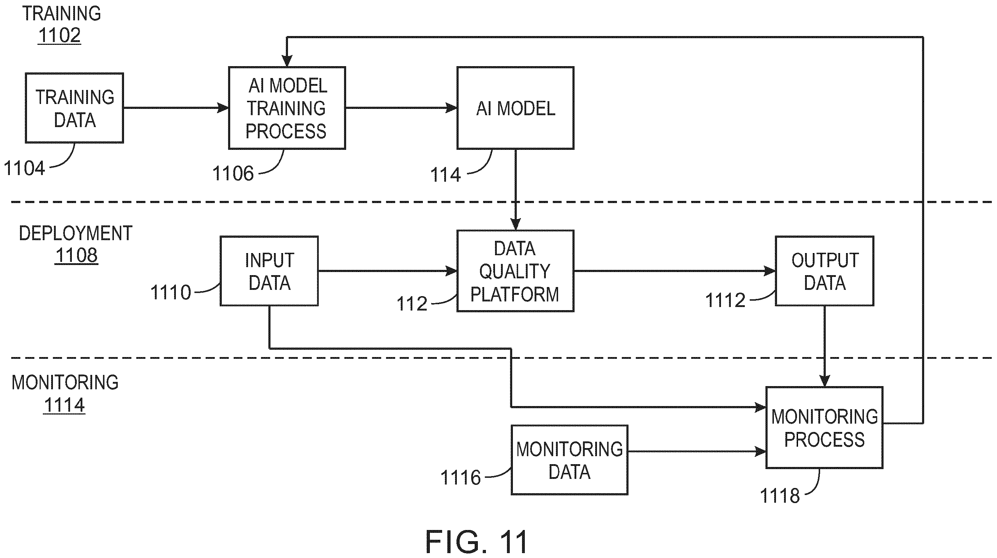

FIG. 11 is a diagram of an example process 1100 for training an artificial intelligence (AI) model. The data quality platform 112 may generate, store, train, and/or use the AI model 114. According to an embodiment, the data quality platform 112 may include the AI model 114 and/or instructions associated with the AI model 114. For example, the data quality platform 112 may include instructions for generating the AI model 114, training the AI model 114, using the AI model 114, etc. According to another embodiment, a system or device other than the data quality platform 112 may be used to generate and/or train the AI model 114. For example, a system or device may include instructions for generating the AI model 114, and/or instructions for training the AI model 114. The system or device may provide a resulting trained AI model 114 to the data quality platform 112 for use.

As shown in FIG. 11, according to an embodiment, the process 1100 may include a training phase 1102, a deployment phase 1108, and a monitoring phase 1114. In the training phase 1102, at operation 1106, the process 1100 may include receiving and processing training data 1104 to generate a trained AI model 114 for determining a data quality of DICOM data. The training data 1104 may include a plurality of training datasets associated with a plurality of radiology devices 102. Each training dataset of the plurality of training datasets may be associated with a radiology device 102. An example training dataset may include DICOM data and an associated data quality of the DICOM data that may be provided as inputs to train the AI model 114.

The training data 1104 may be generated, received, or otherwise obtained from internal and/or external resources. For example, DICOM data may be obtained from the radiology device 102, the data source 104, the data ingestion system 106, and/or the database 110. The training data may identify the data ingestion applications 108 that processed the DICOM data at various stages of data ingestion.

Generally, the AI model 114 may include a set of variables (e.g., nodes, neurons, filters, or the like) that are tuned (e.g., weighted, biased, or the like) to different values via the application of the training data 1104. According to an embodiment, the training process at operation 1106 may employ supervised, unsupervised, semi-supervised, and/or reinforcement learning processes to train the AI model 114. According to an embodiment, a portion of the training data 1104 may be withheld during training and/or used to validate the trained AI model 114.

For supervised learning processes, the training data 1104 may include labels or scores that may facilitate the training process by providing a ground truth. For example, the labels or scores may indicate a data quality of the DICOM data. Training may proceed by feeding a training dataset including DICOM data and an associated data quality from the training data 1104 into the AI model 114. The AI model 114 may have variables set at initialized values (e.g., at random, based on Gaussian noise, based on pre-trained values, or the like). The AI model 114 may output a data quality of DICOM data based on the DICOM data being input to the AI model 114. The output may be compared with the corresponding label or score (e.g., the ground truth) indicating the data quality, which may then be back-propagated through the AI model 114 to adjust the values of the variables. This process may be repeated for a plurality of samples at least until a determined loss or error is below a predefined threshold. According to an embodiment, some of the training data 1104 may be withheld and used to further validate or test the trained AI model 114.

For unsupervised learning processes, the training data 1104 may not include pre-assigned labels or scores to aid the learning process. Instead, unsupervised learning processes may include clustering, classification, or the like, to identify naturally occurring patterns in the training data 1104. As an example, DICOM data may be clustered into groups based on identified similarities and/or patterns. K-means clustering or K-Nearest Neighbors may also be used, which may be supervised or unsupervised. Combinations of K-Nearest Neighbors and an unsupervised cluster technique may also be used. For semi-supervised learning, a combination of training data 1104 with pre-assigned labels or scores and training data 1104 without pre-assigned labels or scores may be used to train the AI model 114.

When reinforcement learning is employed, an agent (e.g., an algorithm) may be trained to make a decision regarding the data quality from the training data 1104 through trial and error. For example, based on making a decision, the agent may then receive feedback (e.g., a positive reward if the prediction was above a predetermined threshold), adjust its next decision to maximize the reward, and repeat until a loss function is optimized.

After being trained, the trained AI model 114 may be stored and subsequently applied by the data quality platform 112 during the deployment phase 1108. For example, during the deployment phase 1108, the trained AI model 114 executed by the data quality platform 112 may receive input data 1110. The input data 1110 may include DICOM data generated by a radiology device 102 and processed by the data ingestion application 108 of the data ingestion system 106. The AI model 114 may provide as output data 1112 a data quality of the DICOM data.

The monitoring data 1116 may include data that identifies a data quality of the DICOM data as determined by an operator. During process 1118, the monitoring data 1116 may be analyzed along with the predicted output data 1112 and input data 1110 to determine an accuracy of the trained AI model 114. According to an embodiment, based on the analysis, the process 1000 may return to the training phase 1102, where at operation 1106 values of one or more variables of the model may be adjusted to improve the accuracy of the AI model 114.

The example process 1000 described above is provided merely as an example, and may include additional, fewer, different, or differently arranged aspects than depicted in FIG. 11.

FIG. 11 describes the training, deployment, and monitoring associated with a trained AI model 114 for determining a data quality of DICOM data. According to an embodiment, one or more other trained AI model 114s may be applied, such as a trained AI model 114 for performing a data validation, for performing anomaly detection, for determining a root cause of the data quality not satisfying a threshold, or the like. Each of the trained AI model 114s may include similar training, deployment, and/or monitoring phases as described above for the trained AI model 114 in FIG. 11, however the particular types of training data, input data, output data, and monitoring data may be different.

Embodiments of the present disclosure shown in the drawings and described above are example embodiments only and are not intended to limit the scope of the appended claims, including any equivalents as included within the scope of the claims. Various modifications are possible and will be readily apparent to the skilled person in the art. It is intended that any combination of non-mutually exclusive features described herein are within the scope of the present invention. That is, features of the described embodiments can be combined with any appropriate aspect described above and optional features of any one aspect can be combined with any other appropriate aspect. Similarly, features set forth in dependent claims can be combined with non-mutually exclusive features of other dependent claims, particularly where the dependent claims depend on the same independent claim. Single claim dependencies may have been used as practice in some jurisdictions require them, but this should not be taken to mean that the features in the dependent claims are mutually exclusive.

## Claims

1. A method (300) comprising:
receiving (302) information related to a data ingestion (402) of Digital Imaging and Communications in Medicine (DICOM) data (412, 416, 420) from a radiology device (102) to a database (110);
determining (304), using a set of data validation rules (414, 418, 422) corresponding to a set of data ingestion stages (404, 406, 408, 410) of the data ingestion (402) of the DICOM data (412, 416, 420) from the radiology device (102) to the database (110), respective data qualities of the DICOM data (412, 416, 420) at the set of data ingestion stages (404, 406, 408, 410) of the data ingestion (402), wherein the data validation rules (414, 418, 422) include one or more of a first data validation rule (602) that is generic to a plurality of imaging modalities of radiology devices (102), a second data validation rule (604, 610, 616) that is specific to an imaging modality of the radiology device (102), and a third data validation rule (606, 608, 612, 614, 618, 620) that is specific to a data ingestion application (108) associated with the data ingestion (402); and
generating (306) a user interface (700, 800) that displays information related to the respective data qualities of the DICOM data (412, 416, 420) at the set of data ingestion stages (404, 406, 408, 410) of the data ingestion (402) based on determining the respective data qualities of the DICOM data (412, 416, 420) at the set of data ingestion stages (404, 406, 408, 410) of the data ingestion (402).

2. The method (300) of claim 1, further comprising:
performing (504) a first data validation using the DICOM data (412, 416, 420) and the first data validation rule (602) that is generic to the plurality of imaging modalities of the radiology devices (102),
wherein the determining (304) the respective data qualities of the DICOM data (412, 416, 420) at the set of data ingestion stages (404, 406, 408, 410) of the data ingestion (402) comprises determining (304) the respective data qualities of the DICOM data (412, 416, 420) at the set of data ingestion stages (404, 406, 408, 410) of the data ingestion (402) based on performing (504) the first data validation.

3. The method (300) of claim of claim 2, further comprising:
performing (506) a second data validation using the DICOM data (412, 416, 420) and the second data validation rule (604, 610, 616) that is specific to the imaging modality of the radiology device (102),
wherein the determining (304) the respective data qualities of the DICOM data (412, 416, 420) at the set of data ingestion stages (404, 406, 408, 410) of the data ingestion (402) comprises determining (304) the respective data qualities of the DICOM data (412, 416, 420) at the set of data ingestion stages (404, 406, 408, 410) of the data ingestion (402) based on performing (506) the second data validation.

4. The method (300) of claim 3, further comprising:
performing (508) a third data validation using the DICOM data (412, 416, 420) and the third data validation rule (606, 608, 612, 614, 618, 620) that is specific to the data ingestion application (108) associated with the data ingestion (402),
wherein the determining (304) the respective data qualities of the DICOM data (412, 416, 420) at the set of data ingestion stages (404, 406, 408, 410) of the data ingestion (402) comprises determining (304) the respective data qualities of the DICOM data (412, 416, 420) at the set of data ingestion stages (404, 406, 408, 410) of the data ingestion (402) based on performing the third data validation.

5. The method (300) of claim 1, further comprising:
determining (902) that a data quality, of the respective data qualities, does not satisfy a threshold;
performing (906) data lineage based on determining that the data quality does not satisfy the threshold; and
determining (908) a root cause of the data quality not satisfying the threshold based on performing the data lineage.

6. The method (300) of claim 5, further comprising:
generating (910) the user interface (700, 800) to display information identifying the root cause.

7. The method (300) of claim 1, wherein the data validation rules (414, 418, 422) include the first data validation rule (602) that is generic to a plurality of imaging modalities of radiology devices (102), the second data validation rule (604, 610, 616) that is specific to an imaging modality of the radiology device (102), and the third data validation rule (606, 608, 612, 614, 618, 620) that is specific to a data ingestion application (108) associated with the data ingestion (402).

8. A device (200) comprising:
a memory (206) configured to store instructions; and
one or more processors (204) configured to execute the instructions to:
receive (302) information related to a data ingestion (402) of Digital Imaging and Communications in Medicine (DICOM) data (412, 416, 420) from a radiology device (102) to a database (110);
determine (304), using a set of data validation rules (414, 418, 422) corresponding to a set of data ingestion stages (404, 406, 408, 410) of the data ingestion (402) of the DICOM data (412, 416, 420) from the radiology device (102) to the database (110), respective data qualities of the DICOM data (412, 416, 420) at the set of data ingestion stages (404, 406, 408, 410) of the data ingestion (402), wherein the data validation rules (414, 418, 422) include a first data validation rule (602) that is generic to a plurality of imaging modalities of radiology devices (102), a second data validation rule (604, 610, 616) that is specific to an imaging modality of the radiology device (102), and a third data validation rule (606, 608, 612, 614, 618, 620) that is specific to a data ingestion application (108) associated with the data ingestion (402); and
generate (306) a user interface (700, 800) that displays information related to the respective data qualities of the DICOM data (412, 416, 420) at the set of data ingestion stages (404, 406, 408, 410) of the data ingestion (402) based on determining the respective data qualities of the DICOM data (412, 416, 420) at the set of data ingestion stages (404, 406, 408, 410) of the data ingestion (402)..

9. The device (200) of claim 8, wherein the one or more processors (204) are further configured to:
perform (504) a first data validation using the DICOM data (412, 416, 420) and the first data validation rule (602) that is generic to the plurality of imaging modalities of the radiology devices (102),
wherein the one or more processors, when determining the respective data qualities of the DICOM data (412, 416, 420) at the set of data ingestion stages (404, 406, 408, 410) of the data ingestion, are further configured to determine the respective data qualities of the DICOM data (412, 416, 420) at the set of data ingestion stages (404, 406, 408, 410) of the data ingestion based on performing (504) the first data validation.

10. The device (200) of claim 9, wherein the one or more processors (204) are further configured to:
perform (506) a second data validation using the DICOM data (412, 416, 420) and the second data validation rule (604, 610, 616) that is specific to the imaging modality of the radiology device (102),
wherein the one or more processors (204), when determining (304) the respective data qualities of the DICOM data (412, 416, 420) at the set of data ingestion stages (404, 406, 408, 410) of the data ingestion (402), are further configured to determine (304) the respective data qualities of the DICOM data (412, 416, 420) at the set of data ingestion stages (404, 406, 408, 410) of the data ingestion (402) based on performing (506) the second data validation.

11. The device (200) of claim 10, wherein the one or more processors (204) are further configured to:
perform (508) a third data validation using the DICOM data (412, 416, 420) and the third data validation rule (606, 608, 612, 614, 618, 620) that is specific to the data ingestion application (108) associated with the data ingestion (402),
wherein the one or more processors (204), when determining (304) the respective data qualities of the DICOM data (412, 416, 420) at the set of data ingestion stages (404, 406, 408, 410) of the data ingestion, are further configured to determine (304) the respective data qualities of the DICOM data (412, 416, 420) at the set of data ingestion stages (404, 406, 408, 410) of the data ingestion based on performing (508) the third data validation.

12. The device (200) of claim 8, wherein the one or more processors (204) are further configured to:
determine (902) that a data quality, of the respective data qualities, does not satisfy a threshold;
perform (906) data lineage based on determining that the data quality does not satisfy the threshold; and
determine (908) a root cause of the data quality not satisfying the threshold based on performing the data lineage.

13. The device (200) of claim 12, wherein the one or more processors (204) are further configured to:
generate (910) the user interface (700, 800) to display information identifying the root cause.

14. The device (200) of claim 8, wherein the one or more processors (204) are further configured to:
determine (902) that a data quality, of the respective data qualities, does not satisfy a threshold,
wherein the information related to the respective data qualities of the DICOM data (412, 416, 420) includes a DICOM tag of a data element of the DICOM data (412, 416, 420) that contributed to the data quality not satisfying the threshold.

15. A non-transitory computer-readable medium (206) storing instructions that, when executed by one or more processors (204), cause the one or more processors (204) to:
receive (302) information related to a data ingestion (402) of Digital Imaging and Communications in Medicine (DICOM) data (412, 416, 420) from a radiology device (102) to a database (110);
determine (304), using a set of data validation rules (414, 418, 422) corresponding to a set of data ingestion stages (404, 406, 408, 410) of the data ingestion (402) of the DICOM data (412, 416, 420) from the radiology device (102) to the database (110), respective data qualities of the DICOM data (412, 416, 420) at the set of data ingestion stages (404, 406, 408, 410) of the data ingestion (402), wherein the data validation rules include a first data validation rule (602) that is generic to a plurality of imaging modalities of radiology devices (102), a second data validation rule (604, 610, 616) that is specific to an imaging modality of the radiology device (102), and a third data validation rule (606, 608, 612, 614, 618, 620) that is specific to a data ingestion application (108) associated with the data ingestion (402); and
generate (306) a user interface (700, 800) that displays information related to the respective data qualities of the DICOM data (412, 416, 420) at the set of data ingestion stages (404, 406, 408, 410) of the data ingestion (402) based on determining the respective data qualities of the DICOM data (412, 416, 420) at the set of data ingestion stages (404, 406, 408, 410) of the data ingestion (402).
